## Europäisches Patentamt

## European Patent Office

## Office européen des brevets

(11) Veröffentlichungsnummer: **0 150 245**
**A1**

## (12) EUROPÄISCHE PATENTANMELDUNG

(21) Anmeldenummer: **84100917.8**

(22) Anmeldetag: **30.01.84**

(51) Int. Cl.⁴: **A 61 B 1/00,** A 61 B 10/00,
A 61 B 17/32

(43) Veröffentlichungstag der Anmeldung: **07.08.85**
**Patentblatt 85/32**

(71) Anmelder: **Storz, Karl, Auf dem Schildrain 39,**
**D-7200 Tuttlingen (DE)**

(72) Erfinder: **Storz, Karl, Auf dem Schildrain 39,**
**D-7200 Tuttlingen (DE)**

(84) Benannte Vertragsstaaten: **AT BE CH DE FR GB IT LI NL**
**SE**

(74) Vertreter: **Wenzel, Joachim, Dipl.-Ing.,**
**Hauptmannsreute 46, D-7000 Stuttgart 1 (DE)**

(54) **Kontaktendoskop.**

(57) Kontaktendoskop für medizinische Zwecke, dessen Frontlinse (1) oder distale Abdeckplatte für das Objektiv mit der zu beobachtenden Stelle in Berührung gebracht wird.

Hierzu ist vorgesehen, daß in einem geringen Abstand vor der Frontlinse ein bandförmiges Messer (9) mit geringer Wandstärke in einer Gleitführung (3) bewegbar ist. Auf diese Weise wird in sehr geringem Abstand vor der Frontlinse eine Probe von genau jenem Teil entnommen, das beobachtet worden ist.

Karl Storz, Tuttlingen

Kontaktendoskop

Die Erfindung betrifft ein Kontaktendoskop nach dem Oberbegriff des Anspruchs 1.

Es besteht die Möglichkeit, ein bekanntes Endoskop mit Panoramasicht durch Zusätze sowohl am Objektiv als auch am Okular so zu verändern, daß sich ein derartiges Kontaktendoskop ergibt. Für den Benutzer entsteht dadurch der Vorteil, daß er sich kein komplettes Kontaktendoskop zu kaufen braucht, sondern ein ohnehin vorhandenes Endoskop mit geringem Aufwand zur Kontaktsicht brauchbar machen kann. Darüber hinaus besteht die Möglichkeit, zunächst eine große Fläche mit kleiner Vergrößerung zu beobachten, und anschließend kann die Beobachtung auf eine sehr starke Vergrößerung im Mikroskopischen-oder Zellenmaßstab umgeschaltet werden und zwar auf eine kleinere Fläche, die für die Untersuchung als noch interessant angesehen wird. Auf diese Weise ist eine sofortige Diagnose eines pathologischen oder Tumorbefundes mit direkter Beobachtung möglich, ohne daß eine Abtragung oder ein

/-2

- 2 -

Eingriff erforderlich ist (DE-OS 30 34 311.4).

Bei einem anderen Kontaktendoskop besteht die Möglichkeit, ebenfalls die Vergrößerung schnell zu ändern. Dies wird hier dadurch erreicht, daß in einem abgezweigten Strahlengang, der zu einem zusätzlichen Okular führt, eine Vergrößerungsoptik mit einer im Vergleich zu dem Endoskop wesentlich stärkeren Vergrößerung angeordnet ist. Dabei ist an der Abzweigstelle ein Schieber quer zu der optischen Achse angeordnet, der eine optische Weiche zur vollständigen Umlenkung des Strahlenganges in den abgezweigten Teil in seiner einen Wirkstellung und in seiner anderen Wirkstellung einen vollständigen Durchlaß der Strahlung für das andere Okular enthält. Auf diese Weise stehen zwei Optiken mit verschiedenen Vergrößerungen jederzeit zur Verfügung, die durch sehr einfaches Umschalten mittels des Schiebers wahlweise in den Strahlengang gebracht werden können (DE-OS 29 48 394.1).

Es sind weiter verschiedene Biopsie-Einrichtungen zur Entnahme von Proben bekannt. Zum Beispiel gibt es eine Gewebestanze mit einem Abstreifer zum Abstreifen der eingezogenen Gewebeteile. Dadurch kann das häufige Herausziehen der Stan-

/-3

- 3 -

ze zum Zwecke der Entnahme des abgestanzten Gewebes oder
der Knochen ganz entfallen. Dabei werden die Gewebestücke
in einem rohrförmigen Schaft untergebracht und dort festgehalten  (DE-GM 77 05 342).

Hierbei kann auch sichergestellt werden, daß die Probestücke in jedem Falle im Bereich des Schaftendes verbleiben, vor allem dadurch, daß sie mit Sicherheit vom Stanzwerkzeug gelöst werden. Hierzu ist innerhalb des Stanzwerkzeuges eine Abstreiferstange mit einem Abstreifer am
distalen Ende angeordnet und durch eine Feder belastet.
Es ist auch/Riegelmechanismus zur Freigabe der federbelasteten Abstreifstange vorgesehen, nachdem diese ihre
äußerste distale Lage erreicht hat (DE-GM 78 01 891.4).

Obwohl es nun möglich ist, mit den eingangs erwähnten bekannten Kontaktendoskopen eine sofortige Diagnose durch
direkte Beobachtung eines pathologischen Tumorgewebes zu
erstellen, besteht doch ein Bedürfnis, aus dem beobachteten Bereich ein Stück abzuschneiden und dieses einer pathologischen Untersuchung zuzuführen.

/-4

- 4 -

Dies ist aber mit den bekannten Biopsie-Zangen nicht möglich, weil die löffelartigen Zangenmaulteile nicht zwischen der Frontlinse des Kontaktendoskopes und dem Gewebe angesetzt werden können.

Der Erfindung liegt die Aufgabe zugrunde, das Kontaktendoskop der im Oberbegriff erwähnten Art so zu verbessern,
daß die Möglichkeit besteht, noch während der Beobachtung
oder zumindest im sofortigen Anschluß daran eine Probe von
der beobachteten Stelle zu entnehmen, ohne das Kontaktendoskop aus der Körperhöhle herausziehen zu müssen.

Zur Lösung dieser Aufgabe sind die kennzeichnenden Merkmale
des Anspruchs 1 vorgesehen. Dadurch kann praktisch noch während der Beobachtung durch das bandförmige Messer in sehr
geringem Abstand vor der Frontlinse eine Probe von genau dem
beobachteten Teil entnommen werden.

In weiterer Ausgestaltung der Erfindung sind die Merkmale
des Anspruchs 2 vorteilhaft. Durch die Öffnung in der Gleitführung kann der Strahlengang einwandfrei geführt werden,
ohne daß die Gleitführung stört. Außerdem hat die Probe

/-5

- 5 -

dann die Form dieser Öffnung, sie wird zwischen der Frontlinse und dem Messer einwandfrei gehalten und kann von
dort auch leicht entnommen werden, indem das Messer nach
dem Herausziehen des Kontaktendoskopes in seine Ausgangslage zurückbewegt wird. Es handelt sich somit um ein guillotineartiges Abtrennen desjenigen Teiles, das sich in der
Öffnung befindet.

Weitere Vorteile und Einzelheiten der Erfindung ergeben
sich aus der nun folgenden Beschreibung einiger Ausführungsformen unter Hinweis auf die Zeichnung. In dieser zeigen:

Fig. 1 eine Seitenansicht auf das gesamte Kontaktendoskop
        nach der Erfindung;

Fig. 2 eine stark vergrößerte Ansicht in Richtung X nach
        der Fig. 3;

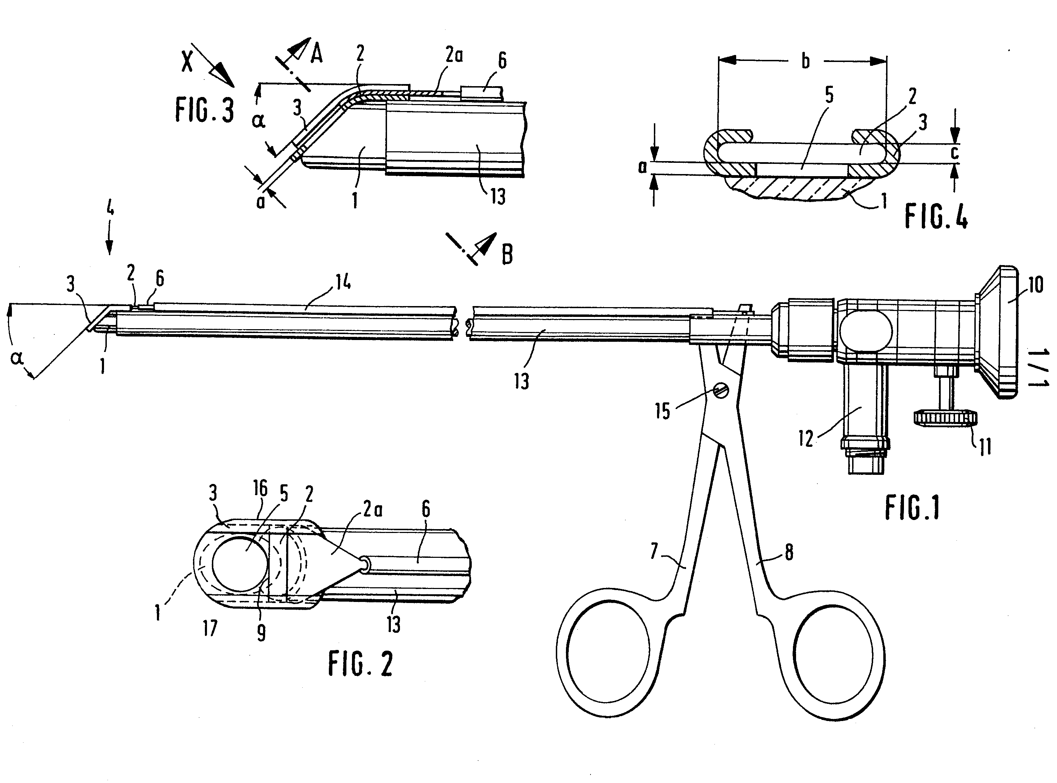

Fig. 3 eine stark vergrößerte Seitenansicht nur auf das
        distale Ende des Kontaktendoskopes nach Fig. 1 und

Fig. 4 einen Schnitt nach der Linie A-B der Fig. 3.

Das Kontaktendoskop nach Fig. 1 zeigt rechts das proximale
Ende mit der Augenmuschel 10, unter der etwas weiter links

/-6

0150245

- 6 -

eine Justierschraube 11 zur Scharfeinstellung sichtbar ist.
Weiter links in Richtung auf das distale Ende 4 sieht man
den Anschluß 12 für einen Lichtleiter bekannter Art. Insoweit handelt es sich um ein herkömmliches Kontaktendoskop,
wie es dem Fachmann bekannt ist und deshalb nicht näher erläutert werden muß. Auch der Strahlengang bis zu der Frontlinse 1 am linken distalen Ende ist dem Fachmann bekannt
und muß daher nicht erläutert werden. Der Blickwinkel $\alpha$
gegenüber der Längsrichtung des Endoskopschaftes kann zum
Beispiel 45 $^o$ betragen, er kann aber noch kleiner sein, zum
Beispiel 30 $^o$ oder auch 0 $^o$ , wobei dann der Strahlengang
im rechten Winkel zur Längsrichtung des Endoskopschaftes 13
zum Okular mit der Augenmuschel 10 geführt wird.

Gemäß der Erfindung ist dieses Kontaktendoskop mit einer
Biopsie-Einrichtung versehen, die nunmehr wie folgt beschrieben wird:

Am linken distalen Ende 4 des Kontaktendoskopes sieht man
vor der Frontlinse 1 und in Berührung mit dieser eine Gleitführung 3, in der ein bandförmiges Messer 2 mit geringer Wandstärke bewegbar ist. Diese Gleitführung 3 folgt oben dem

/-7

0150245

- 7 -

Winkel α und ist am Ende des Endoskopschaftes 13 befestigt. Weiter rechts kommt das bandförmige Messer 2 aus der Gleitführung 3 heraus und ist an einer Betätigungsstange 6 befestigt, die in einem Führungsrohr 14 liegt, das parallel zum Endoskopschaft 13 für den Strahlengang angeordnet ist.

Weiter rechts ist die Betätigungsstange 6 mit der bewegbaren Zangenhälfte 7 so verbunden, daß die Betätigungsstange 6 in Längsrichtung des Endoskopes durch die beiden Hälften der Zange 7, 8 hin- und herbewegbar ist. Die bewegbare Zangenhälfte 7 ist, wie ersichtlich, um den Drehpunkt 15 schwenkbar.

Betätigungszangen dieser Art sind dem Fachmann grundsätzlich bekannt und müssen daher nicht im einzelnen beschrieben werden. Es könnte auch eine andere Betätigungseinrichtung vorgesehen sein, durch die die Betätigungsstange 6 bewegt wird. Fig. 1 zeigt die geöffnete Stellung der Zange, bei der sich das bandförmige Messer 2 in seiner zurückgezogenen Nichtgebrauchslage befindet. Wenn die bewegliche Zangenhälfte 7 nach rechts an die Zangenhälfte 8 gedrückt wird, dann wird

/-8

die Stange 6 nach links geschoben, wie dem Fachmann ohne
weiteres verständlich ist.

Fig. 2 zeigt in stark vergrößerndem Maßstab die Ansicht in
Richtung X gemäß der Fig. 3, welche eine vergrößerte Darstellung des distalen Endes nach der Fig. 3 beinhaltet. Dadurch ergibt sich in Fig. 2 eine Draufsicht auf die Gleitführung 3. In der Mitte sieht man eine Öffnung 5, durch die
die Frontlinse 1 sichtbar ist. Weiter rechts erkennt man die
Schneidkante 9 des bandförmigen Messers 2, dessen Breite b
nach Fig. 4 zur Stange 6 hin abnimmt. Durch diese Maßnahme
paßt sich das distale Ende des Endoskopes den Außenabmessungen besser an, da die Stange 6 gegenüber dem Endoskopschaft
13 für den Strahlengang einen sehr viel kleineren Durchmesser hat. Dieser Übergangsbereich des Messers 2 ist mit 2 a
bezeichnet. Er liegt in dieser Stellung des Messers 2 im
wesentlichen außerhalb der Gleitführung 3.

In diese Ansicht sind die Außenkonturen der Gleitführung 3
bevorzugt als Oblong gehalten, d. h., daß die Seitenkanten
16 und 17 zueinander parallele Geraden sind, welche durch
einen Radius miteinander verbunden sind, der dem halben
Abstand der beiden Geraden zueinander entspricht.

0150245

- 9 -

Fig. 3 zeigt, daß die Gleitführung 3 in einem Bogen um den Winkel α herumgeführt und an dem Endoskopschaft 13 sowie an der Frontlinse 1 befestigt ist. Im Bereich des Messers 2 ist die Gleitführung 3 geschnitten dargestellt, um zu zeigen, wie weit das Messer in diesem zurückgezogenen Falle liegt. Das Messer 2 ist nämlich elastisch und ist in dieser Ruhestellung bereits um den Winkel α in der Führung 3 gebogen. Der Abstand des Messers 2 von der Frontlinse 1 ist mit a bezeichnet und sehr gering. Er entspricht etwa der Wandstärke der Führung 3, wie man der Fig. 4 entnehmen kann. Es handelt sich um einen geringen Bruchteil eines Millimeters. Wenn zum Beispiel das Messer 2 eine Stärke von 0,10 mm hat, was schon verhältnismäßig stark ist, dann ist der Abstand a ebenfalls nur etwa 0,1 mm stark. Die gesamte Gleitführung 3 hat dann vielleicht eine Stärke von 0,3 mm. Es ist aber bekannt, daß zum Beispiel Rasierklingen eine noch weit geringere Wandstärke haben und ebenfalls elastisch sind. Im vorliegenden Falle handelt es sich aber um ein bandförmiges Messer 2, dessen Elastizität oder Biegsamkeit noch größer ist als die einer Rasierklinge.

Fig. 4 stellt einen Schnitt nach der Linie A-B der Fig. 3 dar. Er geht somit durch die Mitte der Öffnung 5, wodurch das Mes-

/-10

ser 2 in seiner vollen Breite b erscheint. Das Messer hat eine Wandstärke c, die etwa in der gleichen Größenordnung ist wie die Wandstärke a der Gleitführung3, die um die Außenkanten des Messers gelegt sind. Hier kann man auch deutlich erkennen, daß die Gleitführung 3 auf der Frontlinse 1 aufliegt, sie kann auch an ihr befestigt sein. Andererseits besteht natürlich auch die Möglichkeit, bei einer anderen Ausführungsform einen Abstandsraum zwischen der Gleitführung 3 und der Frontlinse 1 zu schaffen, wozu hier Abstandshalter zwischen der Gleitführung 3 und der Frontlinse 1 angeordnet sein könnten.

Die Wandstärke der abgeschnittenen Probe richtet sich nämlich nach dem Abstand a zwischen dem Messer 2 und der Frontlinse 1. Da es sich hier um ein Kontaktendoskop handelt, tritt nämlich das zu beobachtende Teil durch die Öffnung 5 und gerät in Kontakt mit der Frontlinse 1, siehe auch Fig. 2. Wenn nun eine Probe entfernt werden soll, werden die Zangenhälften 7,8 zusammengedrückt, wodurch das Messer 2 über die Betätigungsstange 6 nach vorn gedrückt wird. Dadurch schneidet die Schneidkante 9 gemäß Fig. 2 eine kreisrunde Scheibe guillotineartig von dem beobachteten Stück ab. Diese Scheibe entspricht der Öffnung 5 und dem Abstand a zwischen dem Messer 2 und der Frontlinse 1.
Danach wird das Endoskop herausgezogen. Durch Zurückführen
der Zange in die Ausgangslage nach Fig. 1 gibt das Messer
die Probe frei, die in der Öffnung 5 liegt, so daß diese
dann sehr leicht entfernt werden kann.

Es besteht auch noch die Möglichkeit, die Gesamtanordnung
in einem ovalen Außenschaft unterzubringen, zumal der erwähnte Endoskopschaft 13 nur den Strahlengang beinhaltet
und nicht die übrigen Teile.

Die Erfindung ist nicht auf die dargestellten oder geschilderten Ausführungsformen beschränkt. Der Fachmann kann vielmehr im Rahmen der Ansprüche Änderungen hiervon ausführen.

Ansprüche

1. Kontaktendoskop für medizinische Zwecke, dessen Frontlinse oder distale Abdeckplatte für das Objektiv mit
   der zu beobachtenden Stelle in Berührung gebracht wird,
   dadurch gekennzeichnet, daß in einem geringen Abstand
   (a) vor der Frontlinse (1) ein bandförmiges Messer (2)
   mit geringer Wandstärke (c) in einer Gleitführung (3)
   bewegbar ist.

2. Kontaktendoskop nach Anspruch 1, dadurch gekennzeichnet, daß die Gleitführung (3) um das gesamte distale
   Ende (4) herumgeführt ist und daß für den Durchgang
   der Strahlung eine entsprechende Öffnung (5) in der
   Führung freigelassen ist.

3. Kontaktendoskop nach Anspruch 1, dadurch gekennzeichnet, daß das bewegbare Messer (2) so elastisch ist,
   daß es bis zu einem Winkel ($\alpha$) von 45 ° gegenüber
   dem Endoskopschaft während der Betätigung in der Gleitführung (3) biegbar ist.

0150245

4. Kontaktendoskop nach Anspruch 1, dadurch gekennzeichnet, daß das bewegbare Messer (2) mit einer Betätigungsstange (6) fest verbunden ist, die durch eine Betätigungseinrichtung (7,8) am proximalen Ende des Kontaktendoskopes in Achsrichtung desselben hin- und herbewegbar ist.

5. Kontaktendoskop nach Anspruch 4, dadurch gekennzeichnet, daß die Betätigungseinrichtung als Betätigungszange (7,8) ausgebildet ist.

6. Kontaktendoskop nach den Ansprüchen 1 oder 3, dadurch gekennzeichnet, daß das bewegbare Messer (2) in seiner Ruhestellung in der Gleitführung (3) um den Winkel ($\alpha$) gebogen ist.

7. Kontaktendoskop nach Anspruch 1, dadurch gekennzeichnet, daß die Breite (b) des Messers (2) im Bereich (2a) zwischen der Gleitführung (3) und der Betätigungsstange (6) in Richtung auf die Betätigungsstange abnimmt.

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl. 3) |
|---|---|---|---|
| Y | DE-A-2 424 749  (OLYMPUS OPTICAL CORP.) <br> * Seite  11, Zeile 9 - Seite 14, Zeile 4; Figuren 8-11 * | 1 | A 61 B    1/00 <br> A 61 B   10/00 <br> A 61 B   17/32 |
| A | | 3,4,6 | |
| | --- | | |
| Y | US-A-2 540 936  (O.C. DANIELS, SR.) <br> * Insgesamt * | 1 | |
| A | | 2,4,7 | |
| | --- | | |
| A | FR-A-2 249 641  (RICHARD WOLF GmbH) <br> * Seite  3,  Zeile  8 - Seite 4, Zeile 20; Figuren 1-6 * | 1 | **RECHERCHIERTE SACHGEBIETE (Int. Cl. 3)** |
| | --- | | |
| Y | FR-A-2 437 194  (J. HAMOU) <br> * Insgesamt * | 1 | A 61 B    1/00 <br> A 61 B   10/00 <br> A 61 B   17/32 |
| | --- | | |
| Y | US-A-3 407 815  (L. ABELSON) <br> * Insgesamt * | 1 | |
| A | | 2-4,6, 7 | |
| | ---             -/- | | |

Der vorliegende Recherchenbericht wurde für alle Patentansprüche erstellt.

| Recherchenort <br> DEN HAAG | Abschlußdatum der Recherche <br> 28-08-1984 | Prüfer <br> ZILLIOX J.M. |
|---|---|---|

## EINSCHLÄGIGE DOKUMENTE

Seite 2

| Kategorie | Kennzeichnung des Dokuments mit Angabe, soweit erforderlich, der maßgeblichen Teile | Betrifft Anspruch | KLASSIFIKATION DER ANMELDUNG (Int. Cl. ³) |
|---|---|---|---|
| A | FR-A-2 338 687  (R. WOLF GmbH) <br> * Seite 4, Zeile 13 - Seite 5, Zeile 7; Figuren 1-5 * | 1,4 | |
| | --- | | |
| A | FR-A-2 479 680  (METALLISATIONS ET TRAITEMENTS OPTIQUES) | 4,5 | |
| | ----- | | |

RECHERCHIERTE
SACHGEBIETE (Int. Cl. ³)

Der vorliegende Recherchenbericht wurde für alle Patentansprüche erstellt.

| Recherchenort <br> DEN HAAG | Abschlußdatum der Recherche <br> 28-08-1984 | Prüfer <br> ZILLIOX J.M. |
|---|---|---|